# EUROPÄISCHE PATENTSCHRIFT

(11) **EP 3 224 358 B1**
(45) Veröffentlichungstag und Bekanntmachung des Hinweises auf die Patenterteilung: **20.02.2019**
(21) Anmeldenummer: 15791655.2
(22) Anmeldetag: 11.11.2015
(51) Int. Cl.: C12N 9/96, C11D 3/384, C11D 3/386

(54) **VERWENDUNG VON MOLKENPROTEINISOLAT IN ENZYMHALTIGEN WASCH- ODER REINIGUNGSMITTELN ZUR ERHÖHUNG DER STABILITÄT VON ENZYMEN**
USE OF WHEY PROTEIN ISOLATE IN ENZYME-CONTAINING DETERGENTS OR CLEANING AGENTS IN ORDER TO INCREASE ENZYME STABILITY
UTILISATION D'ISOLAT DE PROTÉINE DE PETIT-LAIT DANS DES AGENTS DE LAVAGE OU DE NETTOYAGE CONTENANT DES ENZYMES AFIN D'AUGMENTER LA STABILITÉ DES ENZYMES

(30) Priorität: 25.11.2014 DE 102014223969
(43) Veröffentlichungstag der Anmeldung: 04.10.2017
(73) Patentinhaber: Henkel AG & Co. KGaA, 40589 Düsseldorf (DE)
(72) Erfinder: HELLMUTH, Hendrik, 64297 Darmstadt (DE); LAUFS, Brian, 41363Jüchen (DE); O'CONNELL, Timothy, 40547 Düsseldorf (DE); KROPF, Christian, 40724 Hilden (DE); SUNDER, Matthias, 40593 Düsseldorf (DE)
(86) Internationale Anmeldenummer: PCT/EP2015/076283
(87) Internationale Veröffentlichungsnummer: WO 2016/083127

(56) Entgegenhaltungen:
- EP-A1- 1 953 216
- WO-A1-2013/090559
- DE-C2- 2 660 932

## Beschreibung

Die Erfindung betrifft die Verwendung von Molkenproteinisolat in enzymhaltigen Wasch- oder Reinigungsmitteln zur Erhöhung der Stabilität von Enzymen sowie ein enzymhaltiges Wasch- oder Reinigungsmittel, insbesondere ein flüssiges Wasch- oder Reinigungsmittel mit verbesserter Enzymstabilität.

Übliche Wasch- oder Reinigungsmittel des Marktes enthalten Tenside zur Entfernung von Schmutz und Flecken. In der Regel werden hierbei Kombinationen aus mehreren Tensiden, insbesondere aus der Gruppe der anionischen, nichtionischen, kationischen und amphoteren Tenside verwendet. Diese Tenside allein sind häufig nicht in der Lage, Schmutz und Flecken hinreichend zu entfernen, so dass in modernen Wasch- oder Reinigungsmitteln weitere Hilfsstoffe eingesetzt werden. Zu diesen weiteren Hilfsstoffen gehören Enzyme verschiedener Arten wie Proteasen, Amylasen, Cellulasen, Mannanasen, Pektatlyasen. Dem Fachmann sind weitere Enzymklassen bekannt. Insbesondere hydrolytische Enzyme wie Proteasen, Amylasen oder Lipasen sind wegen ihrer unmittelbar reinigenden Wirkung Bestandteil zahlreicher Textil- oder Geschirrreinigungsmittel.

Die für den Endverbraucher entscheidende Reinigungswirkung der in Wasch- oder Reinigungsmitteln eingesetzten Enzyme wird neben der Enzymstruktur in wesentlichem Maße auch durch die Art der Konfektionierung dieser Enzyme und ihrer Stabilisierung gegen Umwelteinflüsse bestimmt.

Wasch- oder reinigungsaktive Enzyme werden sowohl in fester als auch in flüssiger Form konfektioniert. Zur Gruppe der festen Enzymzubereitungen zählen insbesondere die aus mehreren Inhaltsstoffen bestehenden Enzymgranulate, die ihrerseits vorzugsweise in feste Wasch- oder Reinigungsmittel eingearbeitet werden. Flüssige oder gelförmige Wasch- oder Reinigungsmittel enthalten im Gegensatz hierzu häufig flüssige Enzymzubereitungen, wobei diese, anders als die Enzymgranulate gegen äußere Einflüsse weit weniger geschützt sind.

Zur Erhöhung der Stabilität derartiger enzymhaltiger flüssiger Wasch- oder Reinigungsmittel wurden eine Reihe unterschiedlicher Schutzmaßnahmen vorgeschlagen. So lehrt beispielsweise die deutsche Patentanmeldung DE 20 38 103 (Henkel) die Stabilisierung von enzymhaltigen Geschirrspülmitteln durch Sacharide, während in dem europäischen Patent EP 646 170 B1 (Procter & Gamble) Propylenglykol zur Enzymstabilisierung in flüssigen Reinigungsmitteln offenbart wird.

Als reversible Proteaseinhibitoren sind im Stand der Technik Polyole, insbesondere Glycerin und 1,2-Propylenglycol beschrieben. Eine entsprechende technische Offenbarung findet sich beispielsweise in der internationalen Anmeldung WO 02/08398 A2 (Genencor).

Die Stabilisierung von Enzymen in wässrigen Reinigungsmitteln durch Calciumsalze wie Calciumformiat, Calciumacetat oder Calciumpropionat beschreibt das US amerikanische Patent 4,318,818 (Procter & Gamble). Salze mehrwertiger Kationen wie Calciumkationen führen jedoch in wässrigen Systemen, insbesondere in manuellen Geschirrspülmitteln häufig zu Trübungen während der Lagerung. Dieser negative Effekt verstärkt sich bei der Lagerung bei tiefen Temperaturen. Dadurch sind die möglichen Einsatzkonzentrationen limitiert, so dass keine ausreichende enzymstabilisierende Wirkung garantiert werden kann.

Eine zweite Gruppe bekannter Stabilisatoren bilden Borax, Borsäuren, Boronsäuren oder deren Salze oder Ester. Darunter sind vor allem Derivate mit aromatischen Gruppen, etwa ortho-, meta- oder para-substituierte Phenylboronsäuren zu erwähnen, insbesondere 4-Formylphenyl-Boronsäure (4-FPBA) beziehungsweise die Salze oder Ester der genannten Verbindungen. Letztgenannte Verbindungen als Enzymstabilisatoren sind beispielsweise offenbart in der internationalen Patentanmeldung WO 96/41859 A1 (Novo Nordisk). Allerdings weisen beispielsweise Borsäuren und Borsäurederivate oftmals den Nachteil auf, dass sie mit anderen Inhaltsstoffen einer Zusammensetzung, insbesondere Wasch- bzw. Reinigungsmittelinhaltsstoffen, unerwünschte Nebenprodukte bilden, so dass diese in den betreffenden Mitteln nicht mehr für den erwünschten Reinigungszweck zur Verfügung stehen oder sogar als Verunreinigung auf dem Waschgut zurückbleiben. Ferner werden Borsäuren bzw. Borate unter Umweltaspekten als nachteilig betrachtet.

WO 2013/0905559 A1 (Ecolab USA) beschreibt Enzym-haltige Zusammensetzungen, welche z.B. Molke als Aktivator-Stabilisator enthalten.

Die DE 26 60 932 C2 (Degussa AG) offenbart die Verwendung Molkenprotein-haltiger Mischungen, insbesondere von Süßmolkenpulver, zur Stabilisierung von Lipasen.

Der vorliegenden Erfindung liegt somit die Aufgabe zugrunde, ein Stabilisierungsmittel für Enzyme bereitzustellen, das die Nachteile des Standes der Technik möglichst weitgehend vermeidet.

Überraschenderweise wurde gefunden, daß Molkenproteinisolat in enzymhaltigen Wasch- oder Reinigungsmitteln eine beträchtliche Erhöhung der Stabilität der enthaltenen Enzyme bewirken kann.

Gegenstand der vorliegenden Erfindung ist daher die Verwendung von Molkenproteinisolat in enzymhaltigen Wasch- oder Reinigungsmitteln zur Erhöhung der Stabilität eines Enzyms vom BLAP-Typ.

Bevorzugtermaßen ist das Wasch- oder Reinigungsmittel ein flüssiges Wasch- oder Reinigungsmittel, vorzugsweise ein wässriges, flüssiges Wasch- oder Reinigungsmittel.

Im Rahmen der vorliegenden Erfindung werden unter flüssigen Mitteln solche verstanden, die unter normalen Anwendungsbedingungen fließfähig sind und deren Viskositäten in einem breiten Rahmen variieren können. Zu den flüssigen Zubereitungen zählen auch gelförmige oder pastöse Mittel, welche gegebenenfalls zusätzliche aus dem Stand der Technik bekannte Verdickungsmittel aufweisen können. In einer weiter bevorzugten Ausführungsform der Erfindung beruhen die flüssigen Mittel auf wässriger Basis, wobei die Mittel auch Anteile an organischen Lösungsmitteln aufweisen können. Dem Fachmann sind entsprechende organische Lösungsmittel, welche in flüssigen, wässerigen Wasch- oder Reinigungsmitteln eingesetzt werden können, aus der Literatur bekannt.

Vorteilhafterweise hat es sich herausgestellt, dass Molkenproteinisolat bereits in relativ geringen Mengen zur Stabilisierung von Enzymen beiträgt.

In einer bevorzugten Ausführungsform der Erfindung ist daher ein Mittel vorgesehen, welches Molkenproteinisolat in Mengen von bis zu 5 Gew.-%, vorzugsweise von 0,01 Gew.-% bis 5 Gew.-% enthält. Insbesondere sind Mittel bevorzugt, welche Molkenproteinisolat in Mengen von 0,01 Gew.-% bis 1,4 Gew.-% mit weiterer Bevorzugung von 0,7 bis 1,4 Gew.-% aufweisen.

Ein erfindungsgemäßes Mittel enthält mindestens ein Enzym aus der Gruppe der bekannten, in Wasch- oder Reinigungsmitteln eingesetzten Subtilisine vom BLAP-Typ. In einer bevorzugten Ausführungsform der Erfindung enthält ein erfindungsgemäßes Mittel mindestens eine Protease, sowie mindestens eine Amylase. Von der Protease aus Bacillus lentus DSM 5483 (WO 91/02792 A1) leiten sich die unter der Bezeichnung BLAP® geführten Varianten ab, die insbesondere in WO 92/21760 A1, WO 95/23221 A1, WO 02/088340 A2 und WO 03/038082 A2 beschrieben werden. Besonders bevorzugtermaßen ist die Protease ein Subtilisin vom BLAP-Typ, also eine aus der alkalischen Protease aus Bacillus lentus weiterentwickelte Protease. Besonders bevorzugt ist eine Protease, die ausgehend von BLAP an Position 99 in der Zählung gemäß BLAP die Aminosäure Glutaminsäure (E) aufweist.

Die in erfindungsgemäßen Mitteln eingesetzten Proteasen stammen entweder ursprünglich aus Mikroorganismen, beispielsweise aus Mikroorganismen der Gattungen Bacillus, Streptomyces, Humicola, oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Fungi.

Für Amylasen können synonyme Begriffe verwendet werden, beispielsweise 1,4-alpha-D-Glucan-Glucanohydrolase oder Glycogenase. Erfindungsgemäß konfektionierbare Amylasen sind vorzugsweise α-Amylasen. Entscheidend dafür, ob ein Enzym eine α-Amylase im Sinne der Erfindung ist, ist deren Fähigkeit zur Hydrolyse von a(1-4)-Glykosidbindungen in der Amylose der Stärke.

Erfindungsgemäß konfektionierbare Amylasen sind beispielsweise die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl ® und von dem Unternehmen Danisco/Genencor unter dem Namen Purastar ® ST erhältlich.

Weiterentwicklungsprodukte dieser α-Amylase sind von dem Unternehmen Novozymes unter den Handelsnamen Duramyl ® und Termamyl ® ultra, von dem Unternehmen Danisco/Genencor unter dem Namen Purastar ® OxAm und von dem Unternehmen Daiwa Seiko Inc., Tokyo, Japan, als Keistase ® erhältlich. Die α-Amylase von Bacillus amyloliquefaciens wird von dem Unternehmen Novozymes unter dem Namen BAN ® vertrieben, und abgeleitete Varianten von der α-Amylase aus Bacillus stearothermophilus unter den Namen BSG ® und Novamyl ®, ebenfalls von dem Unternehmen Novozymes. Des Weiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl ® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT ® und Stainzyme ® oder Stainzyme ultra ® bzw. Stainzyme plus ®, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO 07/079938, auf deren Offenbarung daher ausdrücklich verwiesen wird, bzw. deren diesbezüglicher Offenbarungsgehalt daher ausdrücklich in die vorliegende Patentanmeldung mit einbezogen wird.

Ein erfindungsgemäß bevorzugtes Molkenproteinisolat ist das gemäß der Offenbarung der EP 2497368 A1, auf die hiermit in vollem Umfang Bezug genommen wird, erhältliche eiweissangereicherte Erzeugnis.

Besonders bevorzugt ist erfindungsgemäß das von der Fa. Sachsenmilch erhältliche Molkenproteinisolat GermanProt9000.

Alle Sachverhalte, Gegenstände und Ausführungsformen, die für erfindungsgemäße Verwendungen beschrieben sind, sind auch auf die nachfolgend genannten Wasch- oder Reinigungsmittel anwendbar. Daher wird an dieser Stelle ausdrücklich auf die Offenbarung an entsprechender Stelle verwiesen, mit dem Hinweis, dass diese Offenbarung auch für die erfindungsgemäßen Wasch- oder Reinigungsmittel gilt.

Ein weiterer Gegenstand der vorliegenden Erfindung ist ein enzymhaltiges Wasch- oder Reinigungsmittel, das dadurch gekennzeichnet ist, dass es Molkenproteinisolat enthält, insbesondere das von der Fa, Sachsenmilch erhältliche Isolat GermanProt9000. Das enthaltene Enzym ist ein Subtilisin vom BLAP-Typ.

Erfindungsgemäß bevorzugte flüssige Wasch- oder Reinigungsmittel enthalten, bezogen auf ihr Gesamtgewicht, zwischen 0,002 und 7,0 Gew.-%, vorzugsweise zwischen 0,02 und 6,0 Gew.-% und insbesondere zwischen 0,1 und 5,0 Gew.-% Protease-Zubereitungen. Besonders bevorzugt werden Wasch- oder Reinigungsmittel, die bezogen auf ihr Gesamtgewicht zwischen 0,2 und 4,0 Gew.-% Protease-Zubereitungen enthalten.

Erfindungsgemäß bevorzugte flüssige Wasch- oder Reinigungsmittel enthalten, bezogen auf ihr Gesamtgewicht, zwischen 0,001 und 5,0 Gew.-%, vorzugsweise zwischen 0,01 und 4,0 Gew.-% und insbesondere zwischen 0,05 und 3,0 Gew.-% Amylase-Zubereitungen. Besonders bevorzugt werden flüssige Wasch- oder Reinigungsmittel, die bezogen auf ihr Gesamtgewicht zwischen 0,07 und 2,0 Gew.-% Amylase-Zubereitungen enthalten.

Das erfindungsgemäße enzymhaltige Wasch- oder Reinigungsmittel ist in einer bevorzugten Ausführungsform ein Flüssigwaschmittel, in einer anderen bevorzugten Ausführungsform ein flüssiges Mittel zur Reinigung harter Oberflächen, insbesondere von Geschirr.

Unter einem Enzym ist im Sinne der vorliegenden Anmeldung ein Protein zu verstehen, das eine bestimmte biokatalytische Funktion ausübt. Unter Protease im Sinne der vorliegenden Anmeldung wird ein Enzym verstanden, welches die Hydrolyse von Peptidbindungen katalysiert und dadurch in der Lage ist, Peptide oder Proteine zu spalten.

Ein Protein ist im Sinne der vorliegenden Anmeldung ein aus den natürlichen Aminosäuren zusammengesetztes, weitgehend linear aufgebautes, zur Ausübung seiner Funktion zumeist eine dreidimensionale Struktur annehmendes Polypeptid. Ein Peptid besteht aus Aminosäuren, die über Peptidbindungen miteinander kovalent verbunden sind. Die Bezeichnung Polypeptid verdeutlicht diesbezüglich den Sachverhalt, dass diese Peptidkette in der Regel aus vielen Aminosäuren besteht, die über Peptidbindungen miteinander verbunden sind. Aminosäuren können in einer L- und einer D-Konfiguration vorliegen, wobei die Aminosäuren, aus denen Proteine bestehen, in der L-Konfiguration vorliegen. Sie werden als proteinogene Aminosäuren bezeichnet. In der vorliegenden Anmeldung werden die proteinogenen, natürlich vorkommenden L-Aminosäuren mit den international gebräuchlichen 1- und 3-Buchstaben-Codes bezeichnet. Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedingungen durch eine Signalpeptidase vom übrigen Protein abgespalten, so dass dieses seine eigentliche katalytische Aktivität ohne die zunächst vorhandenen N-terminalen Aminosäuren ausübt. Pro-Proteine sind inaktive Vorstufen von Proteinen. Deren Vorläufer mit Signalsequenz werden als Prä-Pro-Proteine bezeichnet. Für technische Anwendungen sind aufgrund ihrer enzymatischen Aktivität die maturen, d.h. reifen Peptide, d.h. die nach ihrer Herstellung prozessierten Enzyme gegenüber den Präproteinen bevorzugt.

Die Proteine können von den sie produzierenden Zellen nach der Herstellung der Polypeptidkette modifiziert werden, beispielsweise durch Anknüpfung von Zuckermolekülen, Formylierungen, Aminierungen, usw. Solche Modifikationen werden als posttranslationale Modifikationen bezeichnet. Diese posttranslationalen Modifizierungen können, müssen jedoch nicht einen Einfluss auf die Funktion des Proteins ausüben.

Proteasen bzw. Enzyme im allgemeinen können durch verschiedene Verfahren, z.B. gezielte genetische Veränderung durch Mutageneseverfahren, weiterentwickelt und für bestimmte Einsatzzwecke oder hinsichtlich spezieller Eigenschaften, beispielsweise katalytische Aktivität, Stabilität, usw., optimiert werden.

Es ist aus dem Stand der Technik weiterhin allgemein bekannt, dass sich vorteilhafte Eigenschaften einzelner Mutationen, z.B. einzelner Punktmutationen, ergänzen können. Eine hinsichtlich bestimmter Eigenschaften bereits optimierte Protease, zum Beispiel hinsichtlich ihrer Stabilität gegenüber Tensiden oder anderen Komponenten, kann erfindungsgemäß zusätzlich weiterentwickelt werden.

Unter Fragmenten werden alle Proteine oder Peptide verstanden, die kleiner sind als natürliche Proteine und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Unter chimären oder hybriden Proteinen sind im Sinne der vorliegenden Anmeldung solche Proteine zu verstehen, deren Sequenz die Sequenzen oder Teilsequenzen von mindestens zwei Ausgangsproteinen umfasst. Die Ausgangsproteine können diesbezüglich aus verschiedenen oder aus demselben Organismus stammen. Chimäre oder hybride Proteine können beispielsweise durch Rekombinationsmutagenese erhalten werden. Der Sinn einer solchen Rekombination kann beispielsweise darin bestehen, mithilfe des heranfusionierten Proteinteils eine bestimmte enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können.

Unter durch Insertionsmutation erhaltene Proteine sind solche Varianten zu verstehen, die durch Einfügen eines Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch die Wirtszelle erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifizierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Ebenso ist unter Derivatisierung die kovalente Bindung an einen makromolekularen Träger zu verstehen, genauso wie auch ein nichtkovalenter Einschluss in geeignete makromolekulare Käfigstrukturen.

In einer weiteren Ausführungsform der Erfindung ist das enzymhaltige Mittel somit dadurch gekennzeichnet, dass das Enzym in dem Mittel als Fragment, Deletionsvariante, chimäres Protein oder Derivat vorliegt, wobei die Protease weiterhin katalytisch aktiv ist.

Im Sinne der vorliegenden Erfindung werden alle Enzyme, Proteine, Fragmente, chimäre Proteine und Derivate, sofern sie nicht explizit als solche angesprochen zu werden brauchen, unter dem Oberbegriff Proteine zusammengefaßt.

Erfindungsgemäße Mittel umfassen alle Arten von enzymhaltigen Mitteln, insbesondere Gemische, Rezepturen, Lösungen etc., deren Enzymstabilität durch Zugabe des oben beschriebenen Molkenproteinisolates verbessert wird. Es kann sich dabei je nach Einsatzgebiet beispielsweise um feste Gemische, beispielsweise Pulver mit gefriergetrockeneten oder verkapselten Proteinen, oder vorzugsweise um gelförmige oder flüssige Mittel handeln.

Insbesondere sind darunter Mittel für die weiter unten ausgeführten Einsatzgebiete zu verstehen. Weitere Einsatzgebiete gehen aus dem Stand der Technik hervor und werden beispielsweise in dem Handbuch "Industrial enyzmes and their applications" von H. Uhlig, Wiley-Verlag, New York, 1998 dargestellt.

In bevorzugten Ausführungsformen der Erfindung ist ein erfindungsgemäßes Mittel dadurch gekennzeichnet, dass es ein Waschmittel, Handwaschmittel, Spülmittel, Handgeschirrspülmittel, Maschinengeschirrspülmittel, Reinigungsmittel, Zahnprothesen- oder Kontaktlinsenpflegemittel, Nachspülmittel, Desinfektionsmittel und insbesondere ein Wäschewaschmittel oder ein Geschirrspülmittel ist.

Zu diesem Erfindungsgegenstand zählen alle denkbaren Wasch- bzw. Reinigungsmittelarten, sowohl Konzentrate als auch unverdünnt anzuwendende Mittel, zum Einsatz im kommerziellen Maßstab, in der Waschmaschine oder bei der Hand-Wäsche beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet.

Ein erfindungsgemäßes Mittel kann sowohl ein Mittel für Großverbraucher oder technische Anwender als auch ein Produkt für den Privatverbraucher sein, wobei alle im Stand der Technik etablierten Wasch- und Reinigungsmittelarten ebenfalls Ausführungsformen der vorliegenden Erfindung darstellen.

Die erfindungsgemäßen Wasch- oder Reinigungsmittel können im Prinzip alle bekannten und in derartigen Mitteln üblichen Inhaltsstoffe enthalten, wobei mindestens ein weiterer Inhaltsstoff in dem Mittel vorhanden ist.

Die erfindungsgemäßen Mittel können insbesondere Buildersubstanzen, oberflächenaktive Tenside, Bleichmittel auf Basis organischer und/oder anorganischer Persauerstoffverbindungen, Bleichaktivatoren, wassermischbare organische Lösungsmittel, Enzyme, Sequestrierungsmittel, Elektrolyte, pH-Regulatoren und weitere Hilfsstoffe wie optische Aufheller, Vergrauungsinhibitoren, Schaumregulatoren sowie Farb- und Duftstoffe sowie Kombinationen hiervon enthalten.

Die erfindungsgemäßen Mittel können ein Tensid oder mehrere Tenside enthalten, wobei insbesondere anionische Tenside, nichtionische Tenside und deren Gemische, aber auch kationische, zwitterionische und amphotere Tenside in Frage kommen.

Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkyl-aminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂-C₁₄-Alkohole mit 3 EO oder 4 EO, C₉-C₁₁-Alkohole mit 7 EO, C₁₃-C₁₅-Alkohole mit 3 EO, 5 EO, 7 EO oder 8 EO, C₁₂-C₁₈-Alkohole mit 3 EO, 5 EO oder 7 EO und Mischungen aus diesen, wie Mischungen aus C₁₂-C₁₄-Alkohol mit 3 EO und C₁₂-C₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind (Talg-) Fettalkohole mit 14 EO, 16 EO, 20 EO, 25 EO, 30 EO oder 40 EO. Insbesondere in Mitteln für den Einsatz in maschinellen Verfahren werden üblicherweise extrem schaumarme Verbindungen eingesetzt. Hierzu zählen vorzugsweise C₁₂-C₁₈-Alkylpolyethylenglykolpolypropylenglykolether mit jeweils bei zu 8 Mol Ethylenoxid- und Propylenoxideinheiten im Molekül. Man kann aber auch andere bekannt schaumarme nichtionische Tenside verwenden, wie zum Beispiel C₁₂-C₁₈-Alkylpolyethylenglykol-polybutylenglykolether mit jeweils bis zu 8 Mol Ethylenoxid- und Butylenoxideinheiten im Molekül sowie endgruppenverschlossene Alkylpolyalkylenglykolmischether. Besonders bevorzugt sind auch die hydroxylgruppenhaltigen alkoxylierten Alkohole, wie sie in der europäischen Patentanmeldung EP 0 300 305 beschrieben sind, sogenannte Hydroxymischether. Zu den nichtionischen Tensiden zählen auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl - die als analytisch zu bestimmende Größe auch gebrochene Werte annehmen kann - zwischen 1 und 10; vorzugsweise liegt x bei 1,2 bis 1,4. Ebenfalls geeignet sind Polyhydroxyfettsäureamide der Formel (III), in der R¹CO für einen aliphatischen Acylrest mit 6 bis 22 Kohlenstoffatomen, R² für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht: 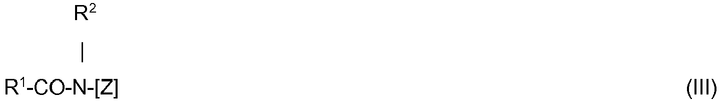 Vorzugsweise leiten sich die Polyhydroxyfettsäureamide von reduzierenden Zuckern mit 5 oder 6 Kohlenstoffatomen, insbesondere von der Glucose ab. Zur Gruppe der Polyhydroxyfettsäureamide gehören auch Verbindungen der Formel (IV), 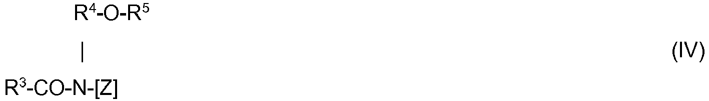 in der R³ für einen linearen oder verzweigten Alkyl- oder Alkenylrest mit 7 bis 12 Kohlenstoffatomen, R⁴ für einen linearen, verzweigten oder cyclischen Alkylenrest oder einen Arylenrest mit 2 bis 8 Kohlenstoffatomen und R⁵ für einen linearen, verzweigten oder cyclischen Alkylrest oder einen Arylrest oder einen Oxy-Alkylrest mit 1 bis 8 Kohlenstoffatomen steht, wobei C₁-C₄-Alkyl- oder Phenylreste bevorzugt sind, und [Z] für einen linearen Polyhydroxyalkylrest, dessen Alkylkette mit mindestens zwei Hydroxylgruppen substituiert ist, oder alkoxylierte, vorzugsweise ethoxylierte oder propoxylierte Derivate dieses Restes steht. [Z] wird auch hier vorzugsweise durch reduktive Aminierung eines Zuckers wie Glucose, Fructose, Maltose, Lactose, Galactose, Mannose oder Xylose erhalten. Die N-Alkoxy- oder N-Aryloxy-substituierten Verbindungen können dann beispielsweise durch Umsetzung mit Fettsäuremethylestern in Gegenwart eines Alkoxids als Katalysator in die gewünschten Polyhydroxyfettsäureamide überführt werden. Eine weitere Klasse bevorzugt eingesetzter nichtionischer Tenside, die entweder als alleiniges nichtionisches Tensid oder in Kombination mit anderen nichtionischen Tensiden, insbesondere zusammen mit alkoxylierten Fettalkoholen und/oder Alkylglykosiden, eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester. Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon. Als weitere Tenside kommen sogenannte Gemini-Tenside in Betracht. Hierunter werden im Allgemeinen solche Verbindungen verstanden, die zwei hydrophile Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, dass die hydrophilen Gruppen einen ausreichenden Abstand haben, damit sie unabhängig voneinander agieren können. Derartige Tenside zeichnen sich im Allgemeinen durch eine ungewöhnlich geringe kritische Micellkonzentration und die Fähigkeit, die Oberflächenspannung des Wassers stark zu reduzieren, aus. In Ausnahmefällen werden unter dem Ausdruck Gemini-Tenside nicht nur derartig "dimere", sondern auch entsprechend "trimere" Tenside verstanden. Geeignete Gemini-Tenside sind beispielsweise sulfatierte Hydroxymischether oder Dimeralkohol-bis- und Trimeralkohol-tris-sulfate und -ethersulfate. Endgruppenverschlossene dimere und trimere Mischether zeichnen sich insbesondere durch ihre Bi- und Multifunktionalität aus. So besitzen die genannten endgruppenverschlossenen Tenside gute Netzeigenschaften und sind dabei schaumarm, so dass sie sich insbesondere für den Einsatz in maschinellen Wasch- oder Reinigungsverfahren eignen. Eingesetzt werden können aber auch Gemini-Polyhydroxyfettsäureamide oder Poly-Polyhydroxyfettsäureamide. Geeignet sind auch die Schwefelsäuremonoester der mit 1 bis 6 Mol Ethylenoxid ethoxylierten geradkettigen oder verzweigten C₇-C₂₁-Alkohole, wie 2-Methylverzweigte C₉-C₁₁-Alkohole mit im Durchschnitt 3,5 Mol Ethylenoxid (EO) oder C₁₂-C₁₈-Fettalkohole mit 1 bis 4 EO. Zu den bevorzugten Aniontensiden gehören auch die Salze der Alkylsulfobernsteinsäure, die auch als Sulfosuccinate oder als Sulfobernsteinsäureester bezeichnet werden, und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈- bis C₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen. Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen. Als weitere anionische Tenside kommen Fettsäure-Derivate von Aminosäuren, beispielsweise von N-Methyltaurin (Tauride) und/oder von N-Methylglycin (Sarkoside) in Betracht. Insbesondere bevorzugt sind dabei die Sarkoside beziehungsweise die Sarkosinate und hier vor allem Sarkosinate von höheren und gegebenenfalls einfach oder mehrfach ungesättigten Fettsäuren wie Oleylsarkosinat. Als weitere anionische Tenside kommen insbesondere Seifen in Betracht. Geeignet sind insbesondere gesättigte Fettsäureseifen, wie die Salze der Laurinsäure, Myristinsäure, Palmitinsäure, Stearinsäure, hydrierten Erucasäure und Behensäure sowie insbesondere aus natürlichen Fettsäuren, zum Beispiel Kokos-, Palmkern- oder Talgfettsäuren, abgeleitete Seifengemische. Zusammen mit diesen Seifen oder als Ersatzmittel für Seifen können auch die bekannten Alkenylbernsteinsäuresalze eingesetzt werden.

Die anionischen Tenside, einschließlich der Seifen, können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Tenside sind in erfindungsgemäßen Mitteln in Mengenanteilen von vorzugsweise 5 Gew.-% bis 50 Gew.-%, insbesondere von 8 Gew.-% bis 30 Gew.-%, enthalten.

Ein erfindungsgemäßes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, insbesondere die durch Oxidation von Polysacchariden beziehungsweise Dextrinen zugänglichen Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 3 000 und 200 000, die der Copolymeren zwischen 2 000 und 200 000, vorzugsweise 30 000 bis 120 000, jeweils bezogen auf freie Säure. Ein besonders bevorzugtes Acrylsäure-Maleinsäure-Copolymer weist eine relative Molekülmasse von 30 000 bis 100 000 auf. Handelsübliche Produkte sind zum Beispiel Sokalan® CP 5, CP 10 und PA 30 der Firma BASF. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/oder einem veresterten Vinylalkohol oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure, wobei Maleinsäure besonders bevorzugt ist, und/oder ein Derivat einer Allylsulfonsäure, die in 2-Stellung mit einem Alkyl- oder Arylrest substituiert ist, sein. Derartige Polymere weisen im Allgemeinen eine relative Molekülmasse zwischen 1 000 und 200 000 auf. Weitere bevorzugte Copolymere sind solche, die als Monomere vorzugsweise Acrolein und Acrylsäure/Acrylsäuresalze beziehungsweise Vinylacetat aufweisen. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt.

Als wasserlösliche anorganische Buildermaterialien kommen insbesondere Alkalisilikate, Alkalicarbonate und Alkaliphosphate, die in Form ihrer alkalischen, neutralen oder sauren Natrium- oder Kaliumsalze vorliegen können, in Betracht. Beispiele hierfür sind Trinatriumphosphat, Tetranatriumdiphosphat, Dinatriumdihydrogendiphosphat, Pentanatriumtriphosphat, sogenanntes Natriumhexametaphosphat, oligomeres Trinatriumphosphat mit Oligomerisierungsgraden von 5 bis 1000, insbesondere 5 bis 50, sowie die entsprechenden Kaliumsalze beziehungsweise Gemische aus Natrium- und Kaliumsalzen. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, allein oder in Mischungen, beispielsweise in Form eines Co-Kristallisats aus den Zeolithen A und X (Vegobond® AX, ein Handelsprodukt der Condea Augusta S.p.A.), bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den Angaben der deutschen Patentschrift DE 24 12 837 bestimmt werden kann, liegt in der Regel im Bereich von 100 bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 22, insbesondere 1,9 bis 4 und y eine Zahl von 0 bis 33 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisitikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Kristalline schichtförmige Silikate der oben angegebenen Formel (I) werden von der Fa. Clariant GmbH unter dem Handelsnamen Na-SKS vertrieben, z.B. Na-SKS-1 (Na₂Si₂₂O₄₅·xH₂O, Kenyait), Na-SKS-2 (Na₂Si₁₄O₂₉·xH₂O, Magadiit), Na-SKS-3 (Na₂Si₈O₁₇·xH₂O) oder Na-SKS-4 (Na₂Si₄O₉·xH₂O, Makatit). Von diesen eignen sich vor allem Na-SKS-5 (α-Na₂Si₂O₅), Na-SKS-7 (β-Na₂Si₂O₅, Natrosilit), Na-SKS-9 (NaHSi₂O₅·3H₂O), Na-SKS-10 (NaHSi₂O₅·3H₂O, Kanemit), Na-SKS-11 (t-Na₂Si₂O₅) und Na-SKS-13 (NaHSi₂O₅), insbesondere aber Na-SKS-6 (δ-Na₂Si₂O₅). In einer bevorzugten Ausgestaltung erfindungsgemäßer Mittel setzt man ein granulares Compound aus kristallinem Schichtsilikat und Citrat, aus kristallinem Schichtsilikat und oben genannter (co-)polymerer Polycarbonsäure, oder aus Alkalisilikat und Alkalicarbonat ein, wie es beispielsweise unter dem Namen Nabion® 15 im Handel erhältlich ist. Buildersubstanzen sind in den erfindungsgemäßen Mitteln vorzugsweise in Mengen bis zu 75 Gew.-%, insbesondere 5 Gew.-% bis 50 enthalten.

Als für den Einsatz in erfindungsgemäßen Mitteln geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, zu denen Perborat, Percarbonat, Persilikat und/oder Persulfat wie Caroat gehören, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Falls ein erfindungsgemäßes Mittel Persauerstoffverbindungen enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Die hydrophil substituierten Acylacetale und die Acyllactame werden ebenfalls bevorzugt eingesetzt. Auch Kombinationen konventioneller Bleichaktivatoren können eingesetzt werden. Derartige Bleichaktivatoren können, insbesondere bei Anwesenheit obengenannter Wasserstoffperoxid-liefernder Bleichmittel, im üblichen Mengenbereich, vorzugsweise in Mengen von 0,5 Gew.-% bis 10 Gew.-%, insbesondere 1 Gew.-% bis 8 Gew.-%, bezogen auf gesamtes Mittel, enthalten sein, fehlen bei Einsatz von Percarbonsäure als alleinigem Bleichmittel jedoch vorzugsweise ganz.

Zusätzlich zu den konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Zu den in den erfindungsgemäßen Mitteln, insbesondere wenn sie in flüssiger oder pastöser Form vorliegen, neben Wasser verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäßen Mitteln in Mengen von vorzugsweise nicht über 20 Gew.-%, insbesondere von 1,2 Gew.-% bis 17 Gew.-%, enthalten. Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Erfindungsgemäße Textilwaschmittel können als optische Aufheller beispielsweise Derivate der Diaminostilbendisulfonsäure beziehungsweise deren Alkalimetallsalze enthalten, obgleich sie für den Einsatz als Colorwaschmittel vorzugsweise frei von optischen Aufhellern sind. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Insbesondere beim Einsatz in maschinellen Verfahren kann es von Vorteil sein, den Mitteln übliche Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

In weiteren Ausführungsformen erfindungsgemäßer Mittel, insbesondere Wasch- oder Reinigungsmittel, werden das zu stabilisierende Enzym und das Molkenproteinisolat beispielsweise mit einzelnen oder mehreren der folgenden Inhaltsstoffe kombiniert: nichtionische, anionische und/oder kationische Tenside, (gegebenenfalls weitere) Bleichmittel, Bleichaktivatoren, Bleichkatalysatoren, Builder und/oder Cobuilder, Säuren, alkalische Substanzen, Hydrotropen, Lösungsmittel, Verdicker, Sequestrierungsmittel, Elektrolyte, optische Aufheller, Vergrauungsinhibitoren, Korrosionsinhibitoren, insbesondere Silberschutzmittel (Silberkorrosionsinhibitoren), Desintegrationshilfsmittel, Soil-Release-Wirkstoffe, Farbtransfer (oder -Übertragungs) -Inhibitoren, Schauminhibitoren, Abrasivstoffe, Farbstoffe, Duftstoffe, Parfüms, antimikrobielle Wirkstoffe, UV-Schutzmittel bzw. -Absorbenzien, Antistatika, Perlglanzmitteln und Hautschutzmitteln, weitere Enzyme wie beispielsweise Protease, Amylase, Cellulase, Hemicellulase, Mannanase, Tannase, Xylanase, Xanthanase, β-Glucosidase, Carrageenase, Oxidase, Oxidoreduktase, Pektin-abbauendes Enzym oder eine Lipase, weitere Stabilisatoren, insbesondere weitere Enzymstabilisatoren, und andere Komponenten, die aus dem Stand der Technik bekannt sind.

In einer weiteren Ausführungsform der Erfindung ist ein erfindungsgemäßes Mittel somit dadurch gekennzeichnet, dass es mindestens eine weitere Komponente enthält, ausgewählt aus der Gruppe bestehend aus Tensiden, Buildern, Säuren, alkalischen Substanzen, Hydrotropen, Lösungsmitteln, Verdickungsmitteln, Bleichmitteln, Farbstoffen, Parfüms, Korrosionsinhibitoren, Sequestriermitteln, Elektrolyten, optischen Aufhellern, Vergrauungsinhibitoren, Silberkorrosionsinhibitoren, Farbübertragungsinhibitoren, Schauminhibitoren, Desintegrationshilfsmittel, Abrasivstoffen, UV-Absorbenzien, Lösungsmitteln, Antistatika, Perlglanzmitteln und Hautschutzmitteln.

Die zu wählenden Inhaltsstoffe wie auch die Bedingungen, unter denen das Mittel eingesetzt wird, wie beispielsweise Temperatur, pH-Wert, lonenstärke, Redox-Verhältnisse oder mechanische Einflüsse, sollten für das jeweilige Reinigungsproblem optimiert sein. So liegen übliche Temperaturen für Wasch- und Reinigungsmittel in Bereichen von 10°C bei manuellen Mitteln über 40°C und 60°C bis hin zu 95° bei maschinellen Mitteln oder bei technischen Anwendungen. Da bei modernen Wasch- und Spülmaschinen die Temperatur meist stufenlos einstellbar ist, sind auch alle Zwischenstufen der Temperatur eingeschlossen. Vorzugsweise werden die Inhaltsstoffe der betreffenden Mittel aufeinander abgestimmt.

In einer weiteren Ausführungsform umfasst ein erfindungsgemäßes Mittel, insbesondere ein Wasch- oder Reinigungsmittel, ferner
- 5 Gew.-% bis 70 Gew.-%, insbesondere 5 Gew.-% bis 30 Gew.-% Tenside und/oder
- 10 Gew.-% bis 65 Gew.-%, insbesondere 12 Gew.-% bis 60 Gew. -% wasserlösliches oder wasserdispergierbares anorganisches Buildermaterial und/oder
- 0,5 Gew.-% bis 10 Gew.-%, insbesondere 1 Gew.-% bis 8 Gew.-%, wasserlösliche organische Buildersubstanzen und/oder
- 0,01 bis 15 Gew.-% feste anorganische und/oder organische Säuren beziehungsweise saure Salze und/oder
- 0,01 bis 5 Gew.-% Komplexbildner für Schwermetalle und/oder
- 0,01 bis 5 Gew.-% Vergrauungsinhibitor und/oder
- 0,01 bis 5 Gew. -% Farbübertragungsinhibitor und/oder
- 0,01 bis 5 Gew.-% Schauminhibitor.

Optional kann das Mittel ferner optische Aufheller umfassen, bevorzugt von 0,01 bis 5 Gew-%.

Die Herstellung erfindungsgemäßer fester Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein einen Extrusionschritt aufweisendes Verfahren bevorzugt.

Zur Herstellung von erfindungsgemäßen Mitteln in Tablettenform, die einphasig oder mehrphasig, einfarbig oder mehrfarbig und insbesondere aus einer Schicht oder aus mehreren, insbesondere aus zwei Schichten bestehen können, geht man vorzugsweise derart vor, dass man alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN, vorzugsweise bei 60 bis 70 kN verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN, insbesondere bei 10 bis 15 kN durchgeführt. Man erhält so problemlos bruchfeste und dennoch unter Anwendungsbedingungen ausreichend schnell lösliche Tabletten mit Bruch- und Biegefestigkeiten von normalerweise 100 bis 200 N, bevorzugt jedoch über 150 N. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind. Ecken und Kanten sind vorteilhafterweise abgerundet. Runde Tabletten weisen vorzugsweise einen Durchmesser von 30 mm bis 40 mm auf. Insbesondere die Größe von eckig oder quaderförmig gestalteten Tabletten, welche überwiegend über die Dosiervorrichtung beispielsweise der Geschirrspülmaschine eingebracht werden, ist abhängig von der Geometrie und dem Volumen dieser Dosiervorrichtung. Beispielhaft bevorzugte Ausführungsformen weisen eine Grundfläche von (20 bis 30 mm) x (34 bis 40 mm), insbesondere von 26x36 mm oder von 24x38 mm auf.

Flüssige beziehungsweise pastöse erfindungsgemäße Mittel in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt. Ausführungsformen der vorliegenden Erfindung umfassen somit alle derartigen festen, pulverförmigen, flüssigen, gelförmigen oder pastösen Darreichungsformen der Mittel, die gegebenenfalls auch aus mehreren Phasen bestehen können sowie in komprimierter oder nicht komprimierter Form vorliegen können. Eine weitere Ausführungsform der Erfindung stellen daher Mittel dar, die dadurch gekennzeichnet sind, dass sie als Einkomponentensystem vorliegen. Solche Mittel bestehen bevorzugt aus einer Phase. Selbstverständlich können erfindungsgemäße Mittel aber auch aus mehreren Phasen bestehen. In einer weiteren Ausführungsform der Erfindung ist das Wasch- oder Reinigungsmittel daher dadurch gekennzeichnet sind, dass es in mehrere Komponenten aufgeteilt ist.

Zu den erfindungsgemäßen festen Darreichungsformen zählen ferner Extrudate, Granulate, Tabletten oder Pouches, die sowohl in Großgebinden als auch portionsweise abgepackt vorliegen können. Alternativ liegt das Mittel als rieselfähiges Pulver vor, insbesondere mit einem Schüttgewicht von 300 g/l bis 1200 g/l, insbesondere 500 g/l bis 900 g/l oder 600 g/l bis 850 g/l.

In einer bevorzugten Ausführungsform der Erfindung liegt das Wasch- oder Reinigungsmittel, in flüssiger, gelförmiger oder pastöser Form vor, insbesondere in Form eines nicht-wässrigen Flüssigwaschmittels oder einer nicht-wässrigen Paste oder besonders bevorzugt in Form eines wässrigen Flüssigwaschmittels oder einer wasserhaltigen Paste.

Das erfindungsgemäße Mittel, insbesondere Wasch- oder Reinigungsmittel, kann in einem Behältnis, vorzugsweise einem luftdurchlässigen Behältnis, verpackt sein, aus dem es kurz vor Gebrauch oder während des Waschvorgangs freigesetzt wird.

Erfindungsgemäße Mittel können auch weitere Proteasen oder andere Enzyme in einer für die Wirksamkeit des Mittels zweckmäßigen Konzentration enthalten. Einen weiteren Gegenstand der Erfindung stellen somit Mittel dar, die ferner eines oder mehrere weitere Enzyme umfassen, wobei prinzipiell alle im Stand der Technik für diese Zwecke etablierten Enzyme einsetzbar sind. Als weitere Enzyme bevorzugt einsetzbar sind alle Enzyme, die in dem erfindungsgemäßen Mittel eine katalytische Aktivität entfalten können, insbesondere Proteasen, Amylasen, Cellulasen, Hemicellulasen, Mannanasen, Tannasen, Xylanasen, Xanthanasen, β-Glucosidasen, Carrageenasen, Oxidasen, Oxidoreduktasen, Pektin-abbauende Enzyme (Pektinasen) oder Lipasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden.

Erfindungsgemäße Mittel enthalten Enzyme vorzugsweise in Gesamtmengen von 1 x 10⁻⁸ bis 5 Gewichts-Prozent bezogen auf aktives Protein. Bevorzugt sind die Enzyme von 0,00001 bis 5 Gew.-%, weiter bevorzugt von 0,0001 bis 2,5 Gew.-%, noch weiter bevorzugt von 0,0001 bis 1 Gew.-% und besonders bevorzugt von 0,0001 bis 0,072 Gew.-% in erfindungsgemäßen Mitteln enthalten, wobei jedes enthaltene Enzym in den genannten Mengenverhältnissen vorliegen kann.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Besonders bevorzugt unterstützen die weiteren Enzyme die Wirkung des Mittels, beispielsweise die Reinigungsleistung eines Wasch- oder Reinigungsmittels, hinsichtlich bestimmter Anschmutzungen oder Flecken. Besonders bevorzugt zeigen die Enzyme synergistische Effekte hinsichtlich ihrer Wirkung gegenüber bestimmter Anschmutzungen oder Flecken, d.h. die in der Mittelzusammensetzung enthaltenen Enzyme unterstützen sich in ihrer Reinigungsleistung gegenseitig. Synergistische Effekte können nicht nur zwischen verschiedenen Enzymen, sondern auch zwischen einem oder mehreren Enzymen und weiteren Inhaltsstoffen des erfindungsgemäßen Mittels auftreten. In einer weiteren bevorzugten Ausführungsform der Erfindung ist das erfindungsgemäße Mittel somit dadurch gekennzeichnet, dass es mindestens ein weiteres Enzym enthält, welches eine Protease, Amylase, Cellulase, Hemicellulase, Mannanase, Tannase, Xylanase, Xanthanase, β-Glucosidase, Carrageenase, Oxidase, Oxidoreduktase, Pektin-abbauendes Enzym oder eine Lipase ist.

Bei dem Vergleich der Leistungen zweier Enzyme, ist zwischen proteingleichem und aktivitätsgleichem Einsatz zu unterscheiden. Insbesondere bei gentechnisch erhaltenen, weitgehend nebenaktivitätsfreien Präparationen ist der proteingleiche Einsatz angebracht. Denn damit ist eine Aussage darüber möglich, ob dieselben Proteinmengen - beispielsweise als Maß für den Ertrag einer fermentativen Produktion - zu vergleichbaren Ergebnissen führen. Klaffen die jeweiligen Verhältnisse von Aktivsubstanz zu Gesamtprotein (die Werte der spezifischen Aktivität) auseinander, so ist ein aktivitätsgleicher Vergleich zu empfehlen, weil hierüber die jeweiligen enzymatischen Eigenschaften verglichen werden. Generell gilt, dass eine niedrige spezifische Aktivität durch Zugabe einer größeren Proteinmenge ausgeglichen werden kann. Hierbei handelt es sich letztlich um eine ökonomische Erwägung.

Die Proteaseaktivität in derartigen Mitteln kann nach der in Tenside, Band 7 (1970), S. 125-132 beschriebenen Methode ermittelt werden. Sie wird entsprechend in PE (Protease-Einheiten) angegeben.

Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist. Die Messung erfolgt bei einer Temperatur von 25°C, bei pH 8,6, und einer Wellenlänge von 410 nm. Die Messzeit beträgt 5 min und das Messintervall 20s bis 60s.

Die in erfindungsgemäßen Mitteln eingesetzten Enzyme stammen entweder ursprünglich aus Mikroorganismen, etwa der Gattungen Bacillus, Streptomyces, Humicola, oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Fungi.

Einen weiteren eigenen Erfindungsgegenstand stellt ein Verfahren zur Reinigung von Textilien oder von harten Oberflächen dar, bei dem in mindestens einem Verfahrensschritt ein erfindungsgemäßes enzymhaltiges Wasch- oder Reinigungsmittel aktiv ist. Das Verfahren zur Reinigung von Textilien oder harten Oberflächen ist demnach dadurch gekennzeichnet, dass in mindestens einem Verfahrensschritt ein erfindungsgemäßes Mittel angewendet wird.

Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind.

Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung dieses Mittels behandelt wird. Das gleiche gilt für Verfahren zur Reinigung von allen anderen Materialien als Textilien, welche unter dem Begriff harte Oberflächen zusammengefasst werden. Alle denkbaren Wasch- oder Reinigungsverfahren können in wenigstens einem der Verfahrensschritte um ein erfindungsgemäßes Mittel bereichert werden, und stellen dann Ausführungsformen der vorliegenden Erfindung dar.

Bevorzugt werden die Enzyme in einer Menge von 40 µg bis 4 g, vorzugsweise von 50 µg bis 3 g, besonders bevorzugt von 100 µg bis 2 g und ganz besonders bevorzugt von 200 µg bis 1 g pro Anwendung eingesetzt.

Einen weiteren eigenen Erfindungsgegenstand stellt ein Verfahren zur Behandlung von Textilrohstoffen oder zur Textilpflege dar, bei dem in mindestens einem Verfahrensschritt ein erfindungsgemäßes enzymhaltiges Wasch- oder Reinigungsmittel aktiv ist.

Hierunter sind Verfahren für Textilrohstoffe, Fasern oder Textilien mit natürlichen Bestandteilen bevorzugt, und ganz besonders für solche mit Wolle oder Seide.

Es kann sich dabei beispielsweise um Verfahren handeln, in denen Materialien zur Verarbeitung in Textilien vorbereitet werden, etwa zur Antifilzausrüstung, oder beispielsweise um Verfahren, welche die Reinigung getragener Textilien um eine pflegende Komponente bereichern. Wegen der oben beschriebenen Wirkung von Proteasen auf natürliche, proteinhaltige Rohstoffe handelt es sich in bevorzugten Ausführungsformen um Verfahren zur Behandlung von Textilrohstoffen, Fasern oder Textilien mit natürlichen Bestandteilen, insbesondere mit Wolle oder Seide.

Die folgenden Beispiele erläutern die Erfindung, ohne sie jedoch darauf einzuschränken:
Beispiel 1: Stabilisierung der Protease I (BLAP mit R99E) in einer Waschmittelmatrix durch Zugabe von Molkeproteinisolat.

Zu einer Waschmittelmatrix (Inhaltsstoffe in Gewichtsprozent, jeweils bezogen auf das gesamte Mittel), enthaltend neben Wasser 5,5 Gew.-% 7-fach ethoxylierten C12/14-Fettakohol,5,3 Gew.-% Natrium-C9-13-Alkylbenzolsulfonat, 4,9 Gew.-% Natrium-C12/14-Fettalkoholethersulfat mit 2 EO, 1,8 Gew.-% Zitronensäure, 3 Gew.-% C12-18-Fettsäure, 0,1 Gew.-% Diethylentriaminpenta-(methylenphosphonsäure)-hepta-Natriumsalz, 1,3 Gew.-% NaOH, 3,6 Gew.-% Ethanol/Glycerin, pH 8,5, mit 1 Gew.% der Protease I wurden unterschiedliche Proteine gegeben. Die Protease-Aktivität wurde mittels eines AAPF-Standardtests, wie in Tenside, Band 7 (1970), S. 125-132. beschrieben, vor Lagerung und dann regelmäßig während der Lagerung bei 30°C bestimmt.

Die Messung erfolgte bei einer Temperatur von 25°C, bei pH 8,6, und einer Wellenlänge von 410 nm. Die Messzeit betrug 5 min und das Messintervall 20s bis 60s.

Dabei wurde das Molkeproteinisolat GermanProt in verschiedenen Konzentrationen sowohl mit den bekannten Molke-Reinproteinen alpha- & beta-Lactalbumin sowie dem Milchprotein Casein als auch mit dem Standardproteinisolat BSA verglichen.

Folgende Restaktivitäten ergaben sich nach 6 Tagen Lagerung:

| | |
|---|---|
| 1,4% GermanProt | 80% |
| 0,7% GermanProt | 66% |
| 0,35% GermanProt | 64% |
| 1,4% β-Lactalbumin | 68% |
| 1,4% α-Lactalbumin | 69% |
| 1,4% Casein | 67% |
| 1,4% BSA | 66% |
| ohne Fremdprotein | 41% |

GermanProt: GermanProt9000, Sachsenmilch
BSA: Bovine serum albumin (Rinderalbumin)

Es wird deutlich, dass die stabilisierende Wirkung des GermanProt erheblich stärker ist als die der bekannten Milch- und Molke-Reinproteine alpha-Lactalbumin, beta-Lactalbumin und Casein und auch die stabilisierende Wirkung anderer Proteinisolate übertrifft.

## Patentansprüche

1. Verwendung von Molkenproteinisolat in enzymhaltigen Wasch- oder Reinigungsmitteln zur Erhöhung der Stabilität eines Subtilisins vom BLAP-Typ.

2. Verwendung nach Anspruch 1, **dadurch gekennzeichnet, dass** das Wasch- oder Reinigungsmittel ein flüssiges Wasch- oder Reinigungsmittel, vorzugsweise ein wässriges, flüssiges Wasch- oder Reinigungsmittel ist.

3. Verwendung nach einem der Ansprüche 1 bis 2, **dadurch gekennzeichnet, dass** das Molkenproteinisolat in Mengen von 0,01 Gew.-% bis 5 Gew.-%, insbesondere in Mengen von 0,01 Gew.-% bis 1,4 Gew.-%, besonders bevorzugt in Mengen von 0,7 bis 1,4 Gew.-% eingesetzt wird.

4. Enzymhaltiges Wasch- oder Reinigungsmittel, **dadurch gekennzeichnet, dass** es Molkenproteinisolat enthält und das enthaltene Enzym ein Subtilisin vom BLAP-Typ ist.

5. Enzymhaltiges Wasch- oder Reinigungsmittel nach Anspruch 4, **dadurch gekennzeichnet, dass** es ein Flüssigwaschmittel ist.

6. Enzymhaltiges Wasch- oder Reinigungsmittel nach Anspruch 4, **dadurch gekennzeichnet, dass** es ein flüssiges Mittel zur Reinigung harter Oberflächen, insbesondere von Geschirr ist.

7. Enzymhaltiges Wasch- oder Reinigungsmittel nach einem der Ansprüche 4 bis 6, **dadurch gekennzeichnet, dass** es Molkenproteinisolat in Mengen von 0,01 Gew.-% bis 5 Gew.-%, insbesondere in Mengen von 0,01 Gew.-% bis 1,4 Gew.-%, besonders bevorzugt in Mengen von 0,7 bis 1,4 Gew.-% enthält.

8. Verfahren zur Reinigung von Textilien oder harten Oberflächen, **dadurch gekennzeichnet, dass** in mindestens einem Verfahrensschritt ein enzymhaltiges Wasch- oder Reinigungsmittel gemäß einem der Ansprüche 4 bis 7 aktiv ist.

## Claims

1. The use of whey protein isolate in enzyme-containing washing or cleaning agents, for increasing the stability of a subtilisin of the BLAP type.

2. The use according to claim 1, **characterized in that** the washing or cleaning agent is a liquid washing or cleaning agent, preferably an aqueous liquid washing or cleaning agent.

3. The use according to one of claims 1 to 2, **characterized in that** the whey protein isolate is used in amounts of from 0.01 wt.% to 5 wt.%, in particular in amounts of from 0.01 wt.% to 1.4 wt.%, particularly preferably in amounts of from 0.7 to 1.4 wt.%.

4. An enzyme-containing washing or cleaning agent, **characterized in that** it contains whey protein isolate and the contained enzyme is a subtilisin of the BLAP type.

5. The enzyme-containing washing or cleaning agent according to claim 4, **characterized in that** it is a liquid washing agent.

6. The enzyme-containing washing or cleaning agent according to claim 4, **characterized in that** it is a liquid agent for cleaning hard surfaces, in particular dishes.

7. The enzyme-containing washing or cleaning agent according to one of claims 4 to 6, **characterized in that** it contains whey protein isolate in amounts of from 0.01 wt.% to 5 wt.%, in particular in amounts of from 0.01 wt.% to 1.4 wt.%, particularly preferably in amounts of from 0.7 to 1.4 wt.%.

8. A method for cleaning textiles or hard surfaces, **characterized in that** an enzyme-containing washing or cleaning agent according to one of claims 4 to 7 is active in at least one method step.

## Revendications

1. Utilisation d'isolat de protéine de petit-lait dans des agents de lavage ou de nettoyage contenant des enzymes afin d'augmenter la stabilité d'une subtilisine de type BLAP.

2. Utilisation selon la revendication 1, **caractérisée en ce que** l'agent de lavage et de nettoyage est un agent de lavage ou de nettoyage liquide, de préférence un agent de lavage ou de nettoyage liquide, aqueux.

3. Utilisation selon l'une des revendications 1 et 2, **caractérisée en ce que** l'isolat de protéine de petit-lait est utilisé en des quantités allant de 0,01 % en poids à 5 % en poids, en particulier en des quantités allant de 0,01 % en poids à 1,4 % en poids, de manière particulièrement préférée en des quantités allant de 0,7 à 1,4 % en poids.

4. Agent de lavage ou de nettoyage contenant des enzymes, **caractérisé en ce qu'**il contient de l'isolat de protéine de petit-lait et que l'enzyme présente est une subtilisine de type BLAP.

5. Agent de lavage ou de nettoyage contenant des enzymes selon la revendication 4, **caractérisé en ce qu'**il s'agit d'un agent de lavage liquide.

6. Agent de lavage ou de nettoyage contenant des enzymes selon la revendication 4, **caractérisé en ce qu'**il s'agit d'un agent liquide servant à nettoyer des surfaces dures, en particulier la vaisselle.

7. Agent de lavage ou de nettoyage contenant des enzymes selon l'une des revendications 4 à 6, **caractérisé en ce qu'**il contient de l'isolat de protéine de petit-lait en des quantités allant de 0,01 % en poids à 5 % en poids, en particulier en des quantités allant de 0,01 % en poids à 1,4 % en poids, de manière particulièrement préférée en des quantités allant de 0,7 à 1,4 % en poids.

8. Procédé de nettoyage de textiles ou de surfaces dure, **caractérisé en ce qu'**un agent de lavage ou de nettoyage contenant des enzymes selon l'une des revendications 4 à 7 est actif à au moins une étape du procédé.
